# EUROPEAN PATENT APPLICATION

(11) **EP 2 308 992 A1**
(43) Date of publication of application: **13.04.2011**
(21) Application number: 08722299.8
(22) Date of filing: 17.03.2008
(51) Int. Cl.: C12Q 1/02, C12Q 1/04

(54) **SCREENING METHOD FOR EFFICIENTLY OBTAINING USEFUL MICROBIAL STRAINS FROM MICROBIAL SAMPLE OBTAINED FROM NATURAL ENVIRONMENT AND REAGENT AND SCREENING KIT TO BE USED THEREFOR**

(71) Applicant: Tsuji, Takashi, Tokyo 192-0919 (JP); Yanagita, Tomotaka, Tokyo 156-0052 (JP); Yamashita, Koji, Tokyo 171-0041 (JP); Kageyama, Kotaro, Tokyo 176-0002 (JP)
(72) Inventor: TSUIJ,Takashi, Tokyo 192-0919 (JP)
(74) Representative: Giovannini, Francesca
(86) International application number: PCT/JP2008/054905
(87) International publication number: WO 2009/116127

(57) **Abstract**

[Problems] To provide a means for efficiently obtaining multiple species of useful microbial strains having high proliferation potency all at once from a microbial sample obtained from a natural environment.

[Means for Solving]

A method for screening for efficiently obtaining useful microbial strains from a sample, which comprises a first step of inoculating a culture medium with the sample potentially containing multiple microbial cells to culture the sample in the presence of a cell enlarger, a second step of, after the first step, introducing an esterase substrate fluorescent stain into the culture medium, a third step of isolating enlarged microbial cells stained in the second step according to a micromanipulation method to produce multiple culture media for establishment each inoculated with a single microbial cell, a fourth step of culturing the microbial cell in each of the culture media for establishment; and a fifth step of selecting culture media for establishment, in which cell proliferation has been confirmed, among the multiple culture media for establishment cultured in the fourth step.

## Description

### TECHNICAL FIELD

The present invention relates to a technique capable of obtaining desired useful microbial strains all at once from microbial samples obtained from natural environments at higher efficiency than by the prior art.

### BACKGROUND ART

In a natural environment, a great variety of microorganisms occur, many of which produce substances useful for human being or decompose harmful substances to provide great benefits.

For instance, among such microorganisms are soil microorganisms such as denitrificans. In recent years, environmental problems such as water system contamination by nitrate nitrogen and generation of N₂O (nitrous oxide) as a greenhouse gas are serious due to a volume use of nitrogenous fertilizers, an increase of imported foodstuff and feedstuff and the like. For solution of such problems, denitrificans are expected to play a significant role. Namely, in rice paddy soil, ammonia (NH₄⁺) derived from nitrogenous fertilizers and domestic wastewater is present. Part of the ammonia is absorbed by rice plant while the rest of the ammonia is first converted by the action of nitrifying bacteria to nitrate ion (N0₃⁻) and finally converted by denitrificans to harmless nitrogen gas (N₂) instead of nitrogen oxides to be returned to the atmosphere. Therefore, rice paddy soil is an excellent farmland with less leaching of nitric acid and less generation of nitrogen oxides such as N₂O in comparison with field soil or grassland soil by virtue of the cooperative action of nitrifying bacteria and denitrificans. At present, however, although various microorganisms that exhibit denitrifying capabilities (denitrificans) under artificial culture conditions are known, reliable identification results concerning the species of denitrificans actually active in rice paddy soil are very few.
Nonpatent Reference 1: Koch, R. (1882). Die Aetiologie der Tuberculose, Berl Klin Wochenschr 19, 221-230.
Patent Reference 1: Japanese Unexamined Patent Publication No. 2000-79000

### DISCLOSURE OF THE INVENTION

### PROBLEMS TO BE SOLVED BY THE INVENTION

As written above, it is both environmentally and industrially valuable to obtain useful microorganisms in a natural environment. Here, as a procedure for obtaining such useful microorganisms, there is a method in which multiple operations each of obtaining a single microbial cell according to a micromanipulation method from a flora of microorganisms (a mass composed of clusters of multiple microorganisms) and transferring it to a test tube (test tube containing a culture medium suitable for the proliferation of desired groups of microorganisms) to let it proliferate are carried out simultaneously using multiple test tubes (micromanipulation method). In a natural environment, however, most microbial cells are dormant (or apparently dead) and only some of them are ready for proliferation. Moreover, even those which are ready for proliferation will proliferate actively only under suitable conditions (that is, when they are placed in suitable culture media) and many of them will die out when they are placed in unsuitable media. For that reason, vital and useful microbial strains with high proliferation potency that are obtainable by a micromanipulation method represent only 1 to 5 % of all the test tubes, with notably poor efficiency.

Then, as another method for more efficiently obtaining vital and useful microbial strains with high proliferation potency, a dilution plating method has been most widely used (see Nonpatent Reference 1). This method involves dispersing microorganisms occurring in a natural environment into sterile water and the like to disassemble them into cells as discrete as possible and then plating them uniformly over an agar plate for proliferation. Subsequently, it is aimed that a number of cells are proliferated from a single cell to form usable colonies. In the dilution plating method, however, microorganisms are not actually separated into single cells, often forming agglomerates each consisting of several to several hundreds of cells, with a result that each colony contains several tens or more of species of cells in mixture. In this case, as the colony proliferates, less competitive microorganisms therein gradually die out and more competitive ones only survive. As a result, even if a large number of colonies occur, many of them tend to be of the same species and the number of species of microorganisms obtained is very limited. Further, the method is useful but suffers a disadvantage that less competitive microorganisms can hardly be obtained.

Thus, in screening useful microbial strains from a flora of microorganisms, since a single microorganism is placed in a test tube with no distinction between microorganisms with or without proliferation potency, according to the former micromanipulation method, the proportion of microorganisms having high proliferation potency is so low in relation to the total number of microorganisms placed in the test tubes that the efficiency in obtaining them can be notably impaired. Also, according to the latter dilution plating method, the probability that less competitive microorganisms die out is so high that the efficiency in obtaining novel useful microbial strains can be impaired, in a manner similar to the former procedure.

As described above, because of the difficulty in efficiently screening useful microbial strains from microbial samples obtained from a natural environment, most of such useful microorganisms have not yet been utilized as a matter of fact. As such, it is a primary object of the present invention to provide a means for efficiently obtaining (method for screening) multiple species of useful microbial strains having high proliferation potency all at once from a microbial sample obtained from a natural environment.

Further, in addition to a very great number of microorganisms present in a natural environment, genetic variations among cells of the same species are so extremely great that microorganisms occurring in the natural environment have an extremely wide variety of genetic and physiological characteristics. Therefore, individual microbial strains require so extremely various types of culture media that it has been difficult to culture such strains in a stable manner for an extended period of time, unless extremely various types of culture media suitable for them can be provided. As a result, even if microorganisms capable of producing useful substances can be obtained from a natural environment such as ocean and soil by a conventional method, the rate of proliferation was so slow that mass culture was prevented and/or that the microorganisms reduced their activities, no longer producing the useful substances, after an extended period of culture. As such, it is a secondary object of the present invention to provide a means for culturing microbial strains obtained from a natural environment for an extended period of time during which they possess high proliferation potency.

### MEANS FOR SOLVING THE PROBLEMS

As a result of intense researches in order to solve the above problems, the present inventors have reached the inventions (1) to (4) below.

The present invention (1) is a method for screening for efficiently obtaining useful microbial strains from a sample, which contains
a first step of inoculating a culture medium with the sample potentially containing multiple microbial cells to culture the sample in the presence of a cell enlarger,
a second step of, after the first step, introducing an esterase substrate fluorescent stain into the culture medium,
a third step of isolating enlarged microbial cells stained in the second step according to a micromanipulation method to produce multiple culture media for establishment each inoculated with a single microbial cell,
a fourth step of culturing the microbial cell in each of the culture media for establishment; and
a fifth step of selecting culture media for establishment, in which cell proliferation has been confirmed, among the multiple culture media for establishment cultured in the fourth step.

The present invention (2) is the method according to the invention (1) wherein the cell enlarger is a pyridonecarboxylic acid-based antibiotic (for example, one or more selected from nalidixic acid, pipemidic acid and piromidic acid).

The present invention (3) is an agent for screening for efficiently obtaining useful microbial strains from a sample potentially containing multiple microbial cells, which contains a pyridonecarboxylic acid-based antibiotic (for example, one or more selected from nalidixic acid, pipemidic acid and piromidic acid) as an active ingredient such that, under conditions where the agent is present in a suitable culture medium, cell division is inhibited to allow the microbial cells to enlarge and one of the enlarged microbial cells is transplanted to a culture medium for establishment to allow cell division to start.

The present invention (4) is a kit for screening for carrying out the method for screening defined in the invention (1) or (2), which includes at least one selected from the group consisting of:
the culture medium used in the first step,
the cell enlarger used in the first step,
the esterase substrate fluorescent stain used in the second step; and
the culture media for establishment used in the third step. It is intended that, even when the culture medium used in the first step contains the cell enlarger in advance, the "kit" contains the culture medium and the cell enlarger. Moreover, since the kit is intended to contain "at least one" of the elements, it may contain the culture medium alone, the cell enlarger alone, the esterase substrate fluorescent stain alone, the culture media for establishment alone, the culture medium plus the cell enlarger, the culture medium plus the esterase substrate fluorescent stain, the culture medium plus culture media for establishment, the culture medium plus the cell enlarger plus the esterase substrate fluorescent stain, the culture medium plus the cell enlarger plus the culture media for establishment, the culture medium plus the cell enlarger plus the esterase substrate fluorescent stain plus the culture media for establishment, the cell enlarger plus the esterase substrate fluorescent stain, the cell enlarger plus the culture media for establishment, the cell enlarger plus the esterase substrate fluorescent stain plus the culture media for establishment, the esterase substrate fluorescent stain plus the culture media for establishment as well as any combination thereof with another constitutional element (for example, an ablation tool etc. used for a micromanipulation method).

Now, definition of each term used in CLAIMS and DESCRIPTION are described. An "esterase substrate fluorescent stain" refers to a stain that is inherently nonfluorescent but will fluoresce by the action of an enzyme, esterase. Therefore, by addition of this stain, since all the living microorganisms (or organisms) have esterase activity, they are stained. A "cell" is a morphological and functional constitutional unit for composing an organism and includes any of eukaryotic cells, prokaryotic cells and archeabacteria. A "sample" is not limited as long as it potentially contains multiple microbial cells, examples of which may include soil, sand, bottom sludge, tissues and interstitial substances of animals, plant tissues, airborne dust, river water, sea water, food, cosmetics and feedstuff. A "cell enlarger" refers to an agent capable of promoting enlargement of cells under conditions where the agent is present and preferably refers to an agent capable of enlarging microbial cells under conditions where an effective amount of the agent is present in a suitable culture medium and allowing cell division of one of the enlarged microbial cells to start when the cell is transplanted in a culture medium for establishment containing no cell enlarger (reversible). An "enlarged microbial cell" refers to a cell that is far larger than a typical microbial cell (1 to 2 µm in size) by observation under a microscope (for example, having a length or size approximately three to four times as large as a typical size). A "strain" refers to a genetically homogeneous population of microorganisms. A "micromanipulation method" refers to a procedure for manipulating microorganisms by manipulating one or more microtools under a microscope. A "micromanipulator" refers to an instrument used for a micromanipulation method.

### EFFECT OF THE INVENTION

According to the invention (1), use of a micromanipulation method and fluorescent stain enables the operation of obtaining multiple enlarged cells one by one from a culture medium containing a cell enlarger to be made in a shorter time length, so that the total time length for transplantation to multiple culture media for establishment to carry out screening may be greatly reduced. As a result, since the time length during which the enlarged cells are applied with (immersed in) the cell enlarger may be approximately the same for all the enlarged cells (for example, average time length +/- two hours), such effects may be provided that (1) insufficient enlargement of useful microorganisms attributable to an insufficient time length for immersion in the cell enlarger may be prevented, with a result that the risk of the useful microorganisms failing to be screened for may be prevented, while (2) suspension of proliferation of useful microorganisms attributable to an excessive time length for immersion in the cell enlarger may be prevented. Thus, for the reasons described above, the present invention (1) lends itself particularly to efficient screening for useful microorganisms. Also, according to the invention (1), useful microbial strains may be efficiently obtained in a short time length as described above, so that the cost, time and labor for obtaining the useful microbial strains (for each useful microorganism) may be reduced.

Further, the invention (1) includes a step of preliminarily culturing a whole flora of microorganisms in a culture medium suitable for desired species of microorganisms in the presence of a cell enlarger, prior to culturing each of the microbial cells in a separate culture medium for establishment. As a result, since microorganisms of a species suited with the culture medium and the cell enlarger, which have higher propagation potency among the species and/or are not in dormancy, can be acquired as enlarged cells, such an effect may be provided that the efficiency in obtaining desired microbial strains may be notably improved by introducing and culturing each of the enlarged cells in a separate culture medium for establishment, in comparison with conventional methods in which each of microorganisms is introduced and cultured in a culture medium for establishment with no distinction between microorganisms with or without proliferation potency (for example, 1 to 5 % according to conventional methods and 20 to 60 % according to the present method. In EXAMPLES, a case of 82/130 = 63 % is presented). Also, since a culture medium and cell enlarger suitable for desired species of microorganisms are used in the preliminary culture, such an effect may be provided that only groups of microorganisms having desired properties (in particular, cultural properties) can be efficiently obtained from a natural environment. Further, since a single cell that has just started proliferation can be obtained, such an effect may be provided that the obtained single cell can surely proliferate into multiple cells. Also, since cells to be established are enlarged, such an effect may be provided that the cells can be easily located under a microscope.

Further, after obtaining useful microbial strains, the same media used for establishment may be directly used so that such states of affairs, due to unsuitability of media as was conventionally the case, that obtained useful microbial strains are so slow in proliferation that mass culture is unable and/or that, after an extended period of culture, useful substances may no longer be produced can be avoided (because microorganisms not preserved in a suitable culture medium will gradually lose proliferation potency during an extended period of time). In other words, since microbial strains obtained by the method according to the invention (1) proliferate easily and quickly in culture media used for establishment, such an effect may be provided that mass culture may be facilitated to enable mass production of useful substances. Further, since microbial strains obtained by the method according to the invention (1) have media that have been found to be very well suitable therefor, such an effect may be provided that preservation under good conditions for an extended period of time may be enabled, or that the microbial strains are highly likely to produce useful substances after an extended period of culture.

Further, since esterase substrate fluorescent stains, which do not bind to nucleic acids, are not toxic in relation to metabolism and only require an innoxious light (for example, a blue light) as an excitation light to fluoresce are used as fluorescent stains, such an effect may be provided that vitality of enlarged cells may be sustained to such a degree that culture for establishment may be possible even after isolation of the enlarged cells.

According to the inventions (2) and (3), since predetermined compounds which act to inhibit cell division are used as cell enlargers, such an effect may be provided that cell enlargement effects may be expected in shorter time lengths in comparison with the cases in which cells are enlarged while allowing cell division, with a result that the efficiency of screening for useful microorganisms may further be increased per unit time.

### BEST MODE FOR CARRYING OUT THE INVENTION

The best mode of the present invention is described below. The technical scope of the present invention is not limited to the best mode. For example, cell enlargers are exemplified as those acting to inhibit cell division and a micromanipulation method is embodied as demonstrated below; however, they are not limited to such embodiments. The method for screening according to the best mode consists of the first to fifth steps described above. Each step is described in detail below.

### First Step

The first step is a step of inoculating a culture medium with a sample potentially containing multiple microbial cells to culture the sample in the presence of a cell enlarger. Here, the order of addition of cell enlargers is not particularly limited and the cell enlargers may be originally contained in the culture media, may be applied to the culture media before inoculation with the sample or may be applied to the culture media after inoculation with the sample. Each element will be described in detail below.

### Cell Enlarger

First, suitable cell enlargers according to the best mode are not particularly limited, as long as (1) at predetermined concentrations, they are disposed to inhibit or suppress cell division to allow microbial cells to enlarge, and (2) when the concentrations are lowered, they will be disposed to lose the action of inhibiting or suppressing cell division, exerting no adverse effect on normal proliferation and other functions (reversible), example of which may include cell division inhibitors for terminating cell division, for example, agents having inhibitory action on enzymes involved in cell division (such as antibiotics and agricultural chemicals). Specific examples of agents having such properties may include pyridonecarboxylic acid-based antibiotics (for example, nalidixic acid, pipemidic acid and piromidic acid). When the cell enlargers are used, cells grow to lengths or sizes approximately three to four times as large as the normal sizes, for example. Here, different types of cell enlargers should be applied to different species of microorganisms. Further, the cell enlargers act as bactericides for microorganisms when their concentrations are high and as cell enlargers for impairing the division function of the microorganisms when the concentrations are lowered (the latter being "cell enlargers" as referred to in the best mode). Thus, by appropriately selecting the type and amount of cell enlargers, selective pressure by the enlargers can be exerted in addition to the species-selective pressure by the culture media. Among the agents mentioned above, for example, nalidixic acid is effective against gram-negative bacteria while pipemidic acid and piromidic acid are effective against gram-positive bacteria. Also, multiple cell enlargers may be used in combination when they are known to have common properties against multiple bacterial species. For instance, in EXAMPLES, for the purpose of obtaining bacteria having denitrification potency (found in both gram-positive and gram-negative bacteria), three antibiotics are used in combination to cover both of these bacteria. The time length for application of the cell enlargers in the first step depends on the type and concentration of the cell enlargers and the species of desired useful microorganisms, but is usually within 24 hours. Also, it is important that the time length for the first step is approximately the same (for example, average time length +/- two hours) for all enlarged cells to be isolated. Here, an "average time length" refers to, for example, an estimated time length during which useful microorganisms desired to be obtained may sufficiently enlarge (for example, determined based on previous data on the same species as the species desired to be obtained). The smaller the deviation from the average time length is, the higher the efficiency in obtaining useful microorganism desired to be obtained is. The reasons for that are (1) insufficient enlargement of useful microorganisms attributable to an insufficient time length of immersion in the cell enlargers may be prevented, with a result that the risk of the useful microorganisms failing to be screened for may be prevented, while (2) suspension of proliferation of useful microorganisms attributable to an excessive time length of immersion in the cell enlargers may be prevented. As to be subsequently described, since a predetermined time length will be needed for color development after addition of a fluorescent stain at the second stage, taking such a time length for color development into consideration, the time length of the first step may be the estimated time length described above minus the time length for color development.

### Culture Medium

Next, culture media to be used are not particularly limited, but it is preferred to select those culture media on which certain species of microorganisms may only grow (that is, media that are suitable for microorganisms desired to be obtained). For instance, when bacteria that carry out photosynthesis and bacteria that do not carry out photosynthesis are to be obtained, a culture medium without an energy source such as glucose (that is, culture medium only with inorganic nutrients) can only allow bacteria that carry out photosynthesis to grow while a culture medium containing an energy source such as glucose can allow bacteria that do not carry out photosynthesis to grow. Those skilled in the art can easily determine which culture medium should be selected when whatever microorganisms are desired to be obtained. For reference, examples of microorganisms desired to be obtained and culture media to be used are listed in Table 1.

**Table 1**

| Microorganisms desired to be obtained | Culture media to be used |
|---|---|
| *Candia* genus | Biggy agar medium |
| *Cryptococcus* genus | Bird seed agar medium |
| *Neisseria* genus | New York City agar medium |
| *Campylobacter* genus | Skirrow's medium |
| *vibrio* genus | TCBS agar medium |
| *Legionella* genus | BCYE agar medium |
| *Yersinia* genus | CIN agar medium |
| *Corynebacterium* genus, *Lactobacillus* genus, *Micrococcus* genus | CLED agar medium |
| *Haemophilus* genus | Chocolate II agar medium with bacitracin |
| *Pseudomonas aeruginosa* | PASA medium |
| Actinomycetes in general | Yeast malt extract agar medium |
| *Streptomyces* genus | Albumin medium |

### Repetitive Operation

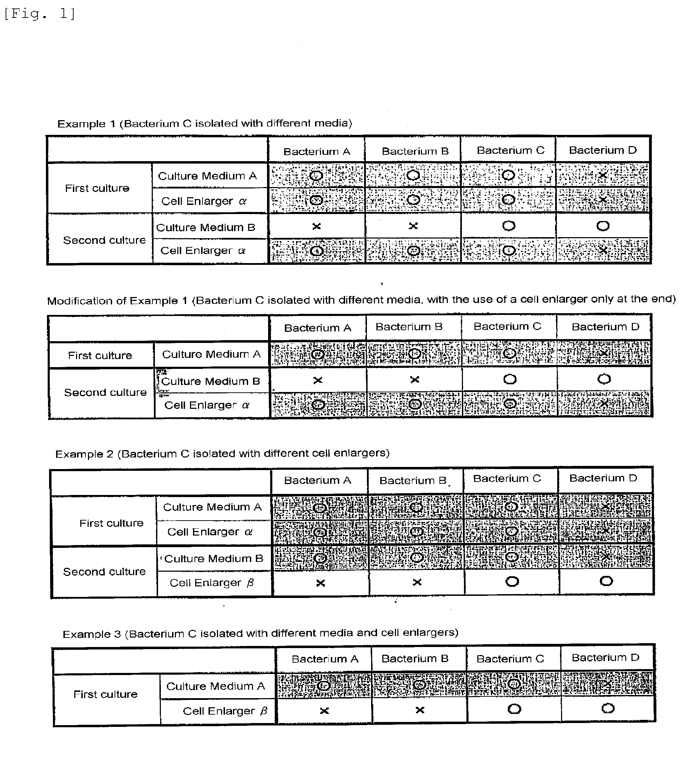

Further, the first step may be repeated multiple times, with different media and/or cell enlargers. For example, at the first step (round 1), a natural sample is cultured in a culture medium in the presence of a cell enlarger to obtain enlarged microorganisms. Next, at the first step (round 2), the enlarged microorganism are cultured in the presence of the same cell enlarger but using another culture medium. Thereby, the enlarged microorganisms obtained through the first step (round 2) must be microorganisms having properties suited with both the culture media of rounds 1 and 2. When this repetitive operation is carried out, it is preferred that the second step to be subsequently described (step of adding an esterase substrate fluorescent stain) be carried out in pair with each round of the first step (for example, a first step (round 1), then a second step, then a first step (round 2), then a second step and so on). Now, referring to Fig. 1, examples of process patterns for obtaining desired microorganisms according to the present steps are described. For the ease of understanding, description is made on a simple model in which four species of microorganisms, namely "Bacterium A" to "Bacterium D" are present in a sample, two culture media, namely, "Culture Medium A" and "Culture Medium B" are used and two cell enlargers, namely, "Cell Enlarger α" and "Cell Enlarger β" are used. In the figure, "o" for a culture medium means that the culture medium is suitable for the growth of a bacterium of interest while "x" for a culture medium means that the culture medium is unsuitable for the growth of a bacterium of interest. "o" for a cell enlarger means that the cell enlarger is an agent exhibiting a cell-enlarging effect on a bacterium of interest at a concentration of use, while "x" for a cell enlarger means that the cell enlarger is an agent that is toxic for a bacterium of interest (a bactericide) at a concentration of use (in this case, the bacterium dies out or is inactivated) or is ineffective against a bacterium of interest (in this case, the bacterium undergoes cell division as usual).

First, Example 1 in Fig. 1 shows an example in which Bacterium C is to be finally isolated by sequentially applying multiple types of media while cell enlargers used remain unchanged in type. Here, first, in the first culture, Selection Medium A for Bacteria A to C is used in the presence of Cell Enlarger α exhibiting a cell-enlarging effect on Bacteria A to C. In this case, Bacteria A to C will proliferate and grow enlarger on the nutrition of the culture medium. On the other hand, Bacterium D will die out or be inactivated instead of proliferating because the culture medium is unsuitable, with a lack of nutrition. In particular, when Cell Enlarger α acts as a toxin, Bacterium D will die out. Thus, in the first culture, Bacteria A to C will be selected. Next, in the second culture, Selection Medium B for Bacteria C and D is used in the presence of Cell Enlarger α exhibiting a cell-enlarging effect on Bacteria A to C. In this case, Bacteria C and D may proliferate on the nutrition of the culture medium. Among them, Bacterium C grows larger because it is suited with Cell Enlarger α while Bacterium D proliferates but remains relatively small because it is not suited with Cell Enlarger α (in addition, since Bacterium D was subjected to an unsuitable medium in the first culture, even if bacteria living through dormancy or the like are present, such bacteria would have been reduced in number or inactivated). Thus, in the second culture, Bacterium C is selected so that only Bacterium C can be isolated.

Here, in Example 1, in order to reliably obtain Bacterium C, a cell enlarger is used at each culture step. However, as long as a cell enlarger is used at the final step (the second culture for Example 1), theoretically, cell enlargers may not be used at part or all of the culture steps along the way. For instance, as a modification of Example 1, an example is shown in which Cell Enlarger α is not used at the first culture step of Example 1. Thus, theoretically, at the first culture step, Bacteria A to D are selected by Culture Medium A while Bacterium D is dormant or die out. Also, cell enlargers are not applied to these bacteria. At the second culture step, Bacteria C and D can grow in Culture Medium B and, since Bacterium D has already died out, only Bacterium C is selected so that finally Bacterium C only grows larger by Cell Enlarger α.

Next, Example 2 is an example in which Bacterium C is finally isolated by sequentially applying multiple types of cell enlargers while culture media used remain unchanged. Here, first, in the first culture, Cell Enlarger α exhibiting a cell-enlarging effect on Bacteria A to C is used in the presence of Culture Medium A suitable for Bacteria A to C. In this case, Bacteria A to C grow larger on the nutrition of the culture medium. On the other hand, Bacterium D dies out or be inactivated instead of growing larger because the culture medium is unsuitable, with a lack of nutrition. In particular, when Cell Enlarger α acts as a toxin, Bacterium D dies out. Next, in the second culture, Cell Enlarger β exhibiting a cell-enlarging effect on Bacteria C and D is used in the presence of Culture Medium A suitable for Bacteria A to C. In this case, Bacteria A to C may proliferate on the nutrition of the culture medium and, among them, Bacteria A and B (1) die out or be inactivated when Cell Enlarger β acts as a toxin, or (2) undergo cell division instead of growing larger (in other words, remain relatively small) when Cell Enlarger β does not act as a toxin. Therefore, only remaining Bacterium C can grow even larger in the culture medium so that only Bacterium C can be isolated.

Next, Example 3 is an example in which media and cell enlargers used are narrowed down to isolate Bacterium C through fewer operations (one in this example). Here, Selection Medium A for Bacteria A to C is used as a culture medium. On the other hand, Cell Enlarger β exhibiting enlarging effect on Bacteria C and D is used as a cell enlarger. Thus, first, when Selection Medium A is used, Bacteria A to C can proliferate on the nutrition of the culture medium. In contrast, Bacterium D dies out or be inactivated instead of proliferating because the culture medium is unsuitable, with a lack of nutrition. Further, when Cell Enlarger β is used, Bacteria C and D can grow larger and, since Bacterium D has died out or been inactivated as described above because the culture medium is unsuitable, with a lack of nutrition, only Bacterium C grows larger on the culture medium. As a result, only Bacterium C can be isolated.

### Second Step

The second step is a step of introducing an esterase substrate fluorescent stain into the culture medium after the first step. Here, during the step, in isolating enlarged microbial cells, use of a stain which only dyes living microorganisms may be used in combination to further facilitate location. Since enlarged cells are weaker in comparison with cells of an ordinary size, however, use of ordinary stains may produce strains which may proliferate only at a reduced rate at a subsequent culture step, may not proliferate or, even if they proliferate, may not produce any useful substances. As such, according to the present method for screening, the stain may need to be an esterase substrate fluorescent stain that will not exert any adverse effects on enlarged cells when the enlarged cells are subsequently cultured. Examples of such stains may include CFDA-AM (5-carboxyfluorescein diacetate, acetoxymethyl ester) and CFDA (carboxyfluorescein diacetate).

Here, the idea of limiting the type of fluorescent stains from the viewpoint of establishment of enlarged cells is novel in itself. This is to be described in detail. First, addition of fluorescent stains after enlargement by application of cell division inhibitors to microbials was known prior to the present application (DVC method, Patent Reference 1). However, it differs from the present invention in that ethidium bromide stain (EB stain) is essentially used as a fluorescent stain, which damages DNA of cells. Under such circumstances, the present inventors attempted to enlarge cells using the DVC method in combination with various stains (a variety of stains including the EB stain) and then isolate and culture the enlarged cells as stained, without success at all for a long time. Then, as a result of intense researches, the present inventors discovered that the reason for no success was that application of a cell enlarger can enlarge cells, but may weaken the cells with conventional stains to render them inapplicable to a subsequent culture. For example, the inventors obtained knowledge that the EB stain described above may be intercalated between the two strands of DNA or may adsorb to certain portions of DNA strands to disturb readout of nucleic acid information, due to which enlarged cells may be damaged. Further, the present inventors obtained knowledge that, for some fluorescent stains, an ultraviolet or near-ultraviolet light must be irradiated for microscopic observation, due to which microbial cells or nucleic acids may be damaged. Bearing such knowledge in mind, the present inventors found that esterase substrate fluorescent stains having such properties that they do not bind to nucleic acids, are not toxic in relation to metabolism and only require an innoxious blue light as an excitation light to fluoresce suffer no such problems as described above and can sustain vitality of enlarged cells to a such a degree that the enlarged cells even after isolation can be cultured. Further, the esterase substrate fluorescent stains only stain living cells and, therefore, are suitable for searching for microbial cells to be subjected to culture. Further, although the esterase substrate fluorescent stains suffer from a disadvantage that, for some of them, emitted fluorescence may be too weak to be easily observed as applied to ordinary cells, even such weak fluorescence can be easily discerned as applied to enlarged cells because the cells are large.

### Third Step

The third step is a step of isolating enlarged microbial cells stained in the second step described above according to a micromanipulation method to produce multiple culture media for establishment each inoculated with a single microbial cell. Each element is described in detail below.

### Micromanipulation Method

First, in quickly separating and obtaining a single enlarged microorganism as a potential candidate for a useful microorganism, a procedure must be carried out under a microscope according to a micromanipulation method. By visually discerning cells during operation of a micromanipulator, microorganisms of desired species can be efficiently selected (in particular, those with characteristic sizes and/or shapes). Further, there are numerous plexiform and filamentous cells in a natural environment and, under circumstances where it is difficult to establish according to a conventional method, a cluster of cells can be separated into individual cells by the micromanipulation method.

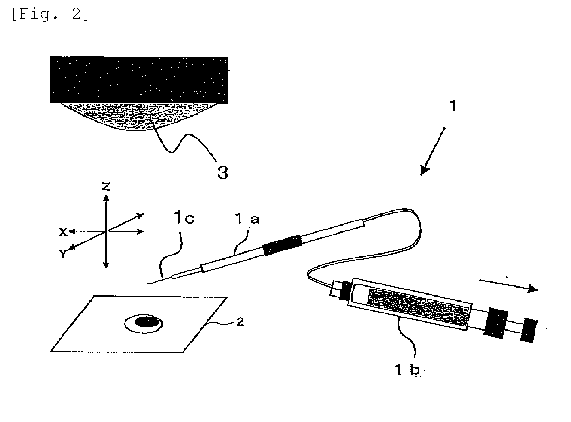

As such, an embodiment of the micromanipulation method is a procedure which uses a holding device capable of holding a single microorganism through physical force such as grasping or suction in separating and obtaining a single enlarged microorganism. Preferred examples of such holding devices may include a micromanipulator capable of sucking a single microorganism with a capillary. Here, such a capillary is preferably made of glass, steel or the like, and has an inner diameter of from 5 to 100 µm and preferably of from 20 to 80 µm. Here, an "inner diameter" means the inner diameter at the tip which may be observed under a microscope. Here, Fig. 2 illustrates a micromanipulator 1 being used to hold a single enlarged microorganism. As illustrated, a capillary holder 1a of the micromanipulator 1 is not secured to a body (not shown) and configured to be movable in microns by oil pressure along the XYZ directions. Also, the capillary holder 1a is coupled to an injection cylinder 1b. By manipulating the injection cylinder 1b, the inside of the capillary holder 1a is held at reduced or increased pressure so that the microorganism may be sucked and released into the holder. To describe a specific procedure for obtaining a microorganism, as illustrated, a sample after culture is placed on a slide glass 2 and then an enlarged microorganism in the sample is searched by observing under a microscope 3. In so doing, it is easy to locate a microorganism since the microorganism is large. After locating such an enlarged microorganism, the capillary holder 1a is appropriately manipulated along the XYZ directions while observing under the microscope so that a capillary 1c at the tip of the capillary holder 1a may contact the enlarged microorganism and then the injection cylinder 1b is manipulated in the direction of the arrow in the drawing so that the microorganism may be sucked into the capillary 1c.

Next, another aspect of the micromanipulation method is a procedure for ablating a single microorganism from a flora of microorganisms. Here, depending on the sample harvested, microorganisms as found may be coupled or coagulated with each other (for a soil sample, empirically approximately 90 % are in such a condition). Here, examples of microtools to be used for ablation are described in detail.

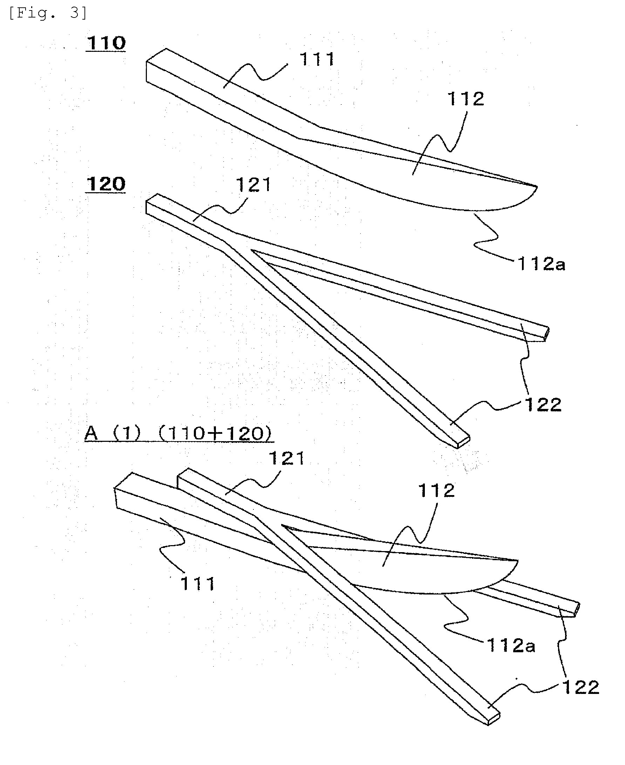

Fig. 3 is a drawing illustrating the structure of an ablation tool A(1) according to such an example. The microtool (ablation tool) A(1) includes an ablator 110, that is, a longitudinal member to which force is directly applied by a micromanipulator and a grasper 120 capable of holding down a microorganism with both arms. Here, the ablator 110 has a fit portion 111 to be fitted to the micromanipulator on one end and an ablation portion 112 for ablating microorganisms from a cluster of numerous filamentous or plexisform microorganisms on the other end. On the other hand, the grasper 120 has a junction 121 to make a joint with the fit portion 111 of the ablator 110 and two flexible legs spread in such a manner that they may catch the ablator 110 approximately in the middle. First, to describe the fit portion 111, the fit portion 111 is not particularly limited as long as it has strength enough to stabilize the ablation tool A(1) when the tool is fitted to the micromanipulator and to withstand ablation operations. Next, the ablation portion 112 is formed with a blade-like ablation area (cutter) 112a at the tip. The ablation portion 112 (the ablation area 112, in particular) is preferably made of a hard material such as steel or glass. For example, the ablation portion 112 can be produced by polishing a sewing needle or fishhook made of high carbon steel with very fine sandpaper. Next, the junction 121 is not particularly limited as long as it is joinable with the fit portion 111 of the ablator 110 by any means. Here, the form of joint between the ablator 110 and the grasper 120 is not particularly limited as along as the ablator 110 is joined in a manner operable in ablation and they may be embodied as integrally formed, as bonded through an adhesive or by welding or soldering or as joined operably by providing a pivot. Next, the two flexible legs 122 may be made of any material as long as they have flexibility enough to press a cluster of microorganisms at two points and may be made of a metal or plastic, for example (the two flexible legs 122 are preferably spread so that the distance between their tips may be wide enough to catch microorganisms, for example, 0.4 mm). Here, the distance between the two flexible legs 122 is variable depending on the size of a cell or a cluster of cells to be handled. In other words, it may be necessary to reduce the distance between the two legs to smaller than the size of the object to be handled so that the cell or the cluster of cells to be handled may be held down by the legs. Therefore, when the size of the object to be handled is 100 µm, for example, the distance between the two flexible legs 122 must be smaller than 100 µm.

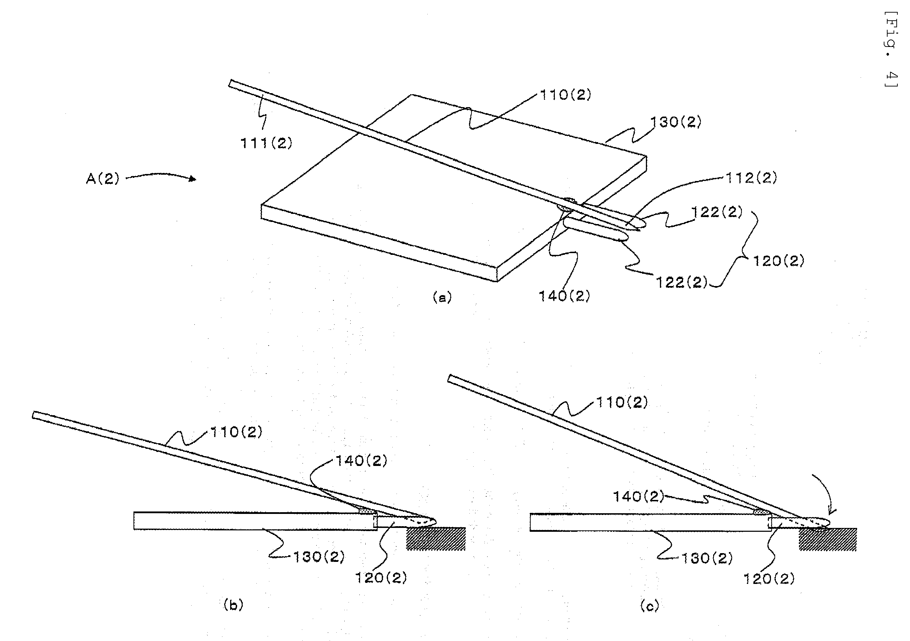

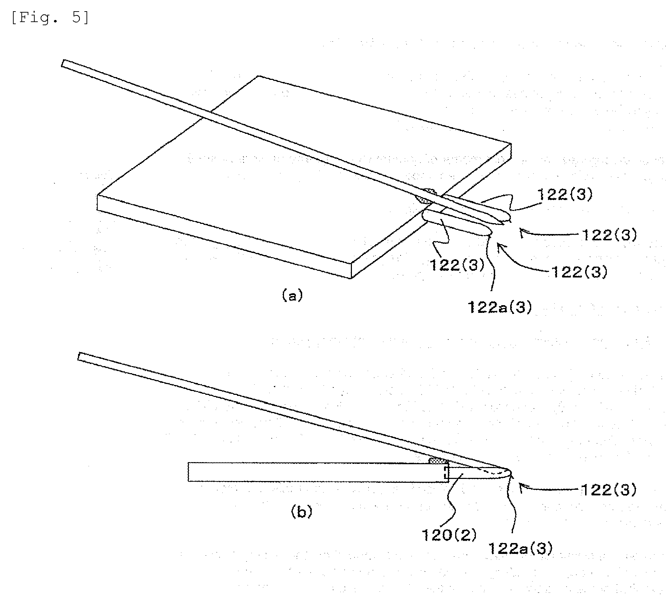

Here, the ablation tool A(1) of Fig. 3 is so embodied that the ablator 110 and the grasper 120 are directly coupled and the grasper 120 is flexible, but it is not limited to such an embodiment. For example, an ablation tool A(2) of Fig. 4 is so embodied that a grasper 120(2) is embedded at an end of a resin base member 130(2) and further an ablator 110(2) is bonded to the base member 130(2) with an elastic adhesive (for example, elastomeric adhesive) 140(2). The base member 130(2) acts only as a fixation member for the ablator 110(2) and the grasper 120(2) and, therefore, it may be smaller in size than illustrated. In this embodiment, the elastic adhesive 140(2) is largely responsible for the flexibility of the ablation tool. Figs. 4(b) and 4(c) are drawings illustrating the relative movement of an ablation portion 112(2) downward in relation to two legs 122(2) when the ablator 110(2) is manipulated by a micromanipulator. Further, an ablation tool A(3) of Fig. 5 is so embodied that each of two legs 122(3) has a protrusion 122a(3) at the tip. Being configured in this manner, it can more reliably immobilize an object to be handled.

To explain how to use the ablation tool A(1), first, any two points of a cluster or mass of interconnected microorganisms are pressed at both the tips of the two flexible legs 122. In this way, slippage of the cluster of microorganisms during operation such as ablation may be prevented and slackness of the cluster of microorganisms may be eliminated to tension the cluster of microorganisms. When the ablation portion 112 is acted on the cluster of microorganisms under this condition, the two flexible legs 112 press on two different points so that slippage of the cluster of microorganisms may be prevented and, in addition, fine ablation may be made with relatively small force because the cluster of microorganisms is tense. The ablation tools A(2) and A(3) are used in the same manner.

### Culture Medium for Establishment

Next, a single enlarged microorganism is typically separated and obtained into a test tube containing a medium for establishment. Here, the culture medium for establishment is a culture medium in which no cell enlarger is present, unlike in the first step. Since it is conceivable that the cell of the enlarged microorganism contains some residual cell enlarger and some cell enlarger is attached on the outside of the cell, it is conceivable that, strictly speaking, it may not be a "culture medium in which no cell enlarger is present" when the enlarged microorganism is introduced into the culture medium. Since the concentration is notably lower in comparison with that in the first step, however, such a case shall be encompassed as a "culture medium in which no cell enlarger is present" as defined in CLAIMS and SPECIFICATION. The number of culture media for establishment is not particularly limited, but is preferably approximately from 50 to 100 for a single operation. Further, the culture medium for establishment is preferably the same or similar in type to the culture medium used in the first step. The medium used in the first step has been confirmed that it is suitable for the enlarged microorganism.

### Time Length for Obtainment

For two reasons to be described below, the shorter the time length required for the third step is, the better. First, the first reason is that the time length for application to cell enlargers in the first step should be approximately the same for all the enlarged cells to be isolated. Namely, as the time lengths for application are approximately the same, (1) insufficient enlargement of useful microorganisms attributable to an insufficient time length for immersion in the cell enlargers may be prevented, with a result that the risk of the useful microorganisms failing to be screened for may be prevented, while (2) suspension of proliferation of useful microorganisms attributable to an excessive time length for immersion in the cell enlargers may be prevented. From this viewpoint, the third step should preferably be carried out within +/- two hours on the basis of the mean value for obtainment of all enlarged cells to be isolated after the end of the first step (that is, within four hours after the start of the isolating operation). The second reason is that when too long a time has passed after addition of a fluorescent stain in the second step, portions other than cells (such as soil) may be stained so that distinction between the enlarged cells and the background may be impossible and, in addition, not only living cells but also dead cells may be stained so that vitality testing may be difficult. From this viewpoint, it should preferably be made within 40 minutes to four hours after addition of a fluorescent stain in the second step. Bearing the two respects described above in mind, it is preferred that the operation of the third step be started 40 minutes after addition of a fluorescent stain in the second step and all the operations be complete within four hours, in case of the timing example described above (the timing described above may vary depending on the type, concentration and the like of the agents and stains used. It should be considered a mere example). Therefore, the time length allowed for one operation for establishment depends on the number of strains to be screened for, but is from one to two minutes per strain for a case of 1,000 strains of interest, for example. Within this time length, microorganisms to be screened for are located and isolated under a microscope (specifically, among enlarged cells, those having characteristics of desired species are discovered and enlarged cells having such characteristics are obtained according to a micromanipulation method). Obtainment of enlarged cells to be screened for in such a short time length can only be realized by a micromanipulation method in combination with a fluorescent stain.

### Fourth Step

The fourth step is a step of culturing microbial cells in multiple culture media for establishment. The step is a well-known, conventional procedure and is carried out for a predetermined period of time (for example, one to five weeks). Thus, when a single microbial cell is captured according to a micromanipulation method under a microscope to transfer it into a test tube containing only a culture medium for establishment to be proliferated, the single large cell is divided into several cells having normal size due to the absence of a cell enlarger. That is, the single large cell starts proliferating and in a few days, one microbial strain may be created as a result of production of a number of cells having the same genes. This strain will proliferate well in the absence of the cell enlargers described above.

### Fifth Step

The fifth step is a step of selecting culture media for establishment, in which cell proliferation has been confirmed, among the multiple culture media for establishment cultured in the fourth step. The step is a well-known, conventional procedure.

### Application

Next, application examples with the use of the present method for screening are described. First, the present method is useful in bioremediation. Bioremediation is a technique intended for clarification and restoration from environmental contamination of soil, groundwater and the like by decomposing and detoxifying contaminants by utilizing actions of microorganisms and the like. Discovery of microorganisms involved in the decomposition and detoxification of such contaminants may be efficiently carried out. For example, there is a possibility that microbial strains capable of eliminating undesired substances in a natural environment, such as PCB and plastics, may be found. Since enlarged microorganisms populating substances desired to be decomposed can be discovered, it is highly possible that such populations of enlarged microorganisms contain microbial cells having promising decomposing potency. Further, these methods are also useful in discovery of microorganisms essential for creating new drugs. In particular, actinomycetes are known to produce a large number of important and useful substances essential for pharmaceuticals. It is, therefore, important to search for new species of actinomycetes. As such, soil or the like in which actinomycetes are contained is placed in a culture medium which allows only the actinomycetes to grow, with addition of a cell division inhibitor, so that only the actinomycetes may grow larger. When a single cell of an actinomycete is obtained among them and cultured according to the present method, various actinomycetes, including unknown ones, may be obtained with high efficiency. Thus, the present method for screening is applicable in a wide variety of industrial fields such as bioremediation of soil, biodesulfurization of petroleum and manufacture of pharmaceuticals and can reliably establish useful microorganisms occurring in the natural world in a short period of time so that they may be obtained in bulk.

### EXAMPLES

It is believed that denitrificans in rice paddy soil suppress contamination of groundwater with nitrate nitrogen due to excessive fertilization and/or suppress generation of N₂O nitrogen oxide having a very strong greenhouse effect. Although a large number of bacteria exhibiting denitrification effects at laboratory levels have been known to exist so far, the number of bacteria found to exhibit denitrification effects actually in rice paddy soil is very limited. This is due to that more competitive bacteria can only be cultured according to conventional methods and that bacteria exhibiting denitrification effects in actual rice paddy soil cannot be identified.

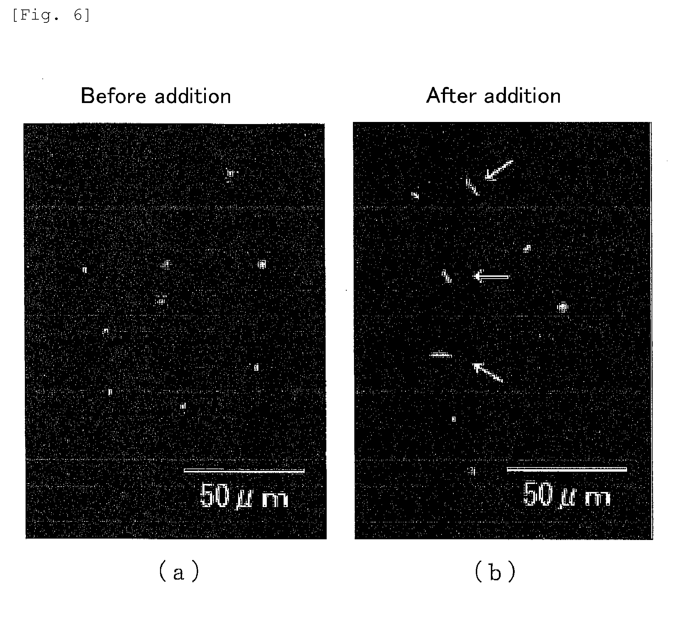

As such, as a laboratory model of rice paddy soil where denitrification is active, a 10 mL vial was filled with slightly less than 10 mL of soil (rice paddy soil from Tanashi Farm of the University of Tokyo), a selective substrate for denitrificans (sodium succinate, approximately 5 g/L) and sodium nitrate (approximately 5 g/L) and the gas phase was completely substituted with argon-acetylene. Culture was then carried out anaerobically at 30°C. After culturing for 24 hours, the substrate (sodium succinate) and sodium nitrate were again added so that the final concentration of each was approximately 5 g/L and simultaneously three cell enlargers, namely nalidixic acid (80 µg/g of soil), pipemidic acid (40 µg/g of soil) and piromidic acid (40 µg/g of soil) were added, followed by further culturing anaerobically at 30°C for 24 hours. A fluorescent stain CFDA-AM (final concentration 50 µM) was added to the cultured product and the product was observed under a fluorescent microscope (excited in blue, irradiated with a blue light) (Fig. 6(b)). In combination, as a comparative example, the fluorescent stain was added and observation was carried out under a microscope with no cell enlargers added (Fig. 6(a)). As a result, in Fig. 6(a) only round or oval microbial cells were observed in the sample, while in Fig. 6(b) elongated and larger cells were also observable (indicated by arrows).

Forty-five minutes after the addition of the fluorescent stain, enlarged cells were isolated by a micromanipulator, observing under a fluorescent microscope, and respectively placed in separate LB liquid media (130 media). The time length required for all the operations from the start of the isolation of the enlarged cells to the transplantation to the LB liquid media to complete was 190 minutes. Thereafter, when these 130 LB liquid media were cultured for one week, 82 strains grew and proliferated. The strains that showed proliferation were cultured in a Giltay liquid medium for one week and assayed for the presence or absence of denitrification effects on the basis of gas generation and discoloration of the culture medium. Fifty strains were then determined to possess denitrification effects. Eight among the 50 strains that were observed with denitrification effects were proliferated and phylogenetically analyzed on the basis of 16S rDNA base sequences of the bacteria. In the example described above, aseptic manipulations were carried out if necessary.

As a result of the phylogenetic analysis, it was determined that they consist of five bacteria as follows: *Stenotrophomonas sp.* (two strains), *Ochrobactrum anthropi* (one strain), *Burkholderia cepacia* (two strains), *Pseudomonas putida* (two strains) and *Pseudomonas sp.* (one strain). The first two of them, that is, *Stenotrophomonas sp.* and *Ochrobactrum anthropi* have not traditionally been supposed to possess denitrification effects, but it has been found anew that they possess denitrification effects. The three others including *Burkholderia cepacia* have traditionally been recognized with denitrification effects as strains cultured in laboratories, and have been easily confirmed with their denitrification effects on a specific sample from rice paddy soil by application of the method according to the present invention.

### BRIEF DESCRIPTION OF THE DRAWINGS

Fig. 1 shows examples of process patterns for obtaining desired microorganisms according to the present method. Here, in order to obtain Bacterium C, three examples are presented in which different media and cell enlargers are used in different combinations.
Fig. 2 is a drawing illustrating a micromanipulator being used to hold a single enlarged microorganism.
Fig. 3 is a drawing illustrating an ablation tool A(1) according to the best mode.
Fig. 4 is a drawing illustrating an ablation tool A(2) according to the best mode.
Fig. 5 is a drawing illustrating an ablation tool A(3) according to the best mode.
Fig. 6 shows photographs showing the effects of cell enlargers on microorganisms in soil in EXAMPLES (Fig. 6(a) before addition, Fig. 6(b) after addition).

### DESIGNATION OF REFERENCE NUMERALS

- 1:: micromanipulator
- 1a:: capillary holder
- 1b:: injection cylinder
- 1c:: capillary
- 2:: slide glass
- 3:: object glass of microscope

## Claims

1. A method for screening for efficiently obtaining useful microbial strains from a sample, which comprises
a first step of inoculating a culture medium with the sample potentially containing multiple microbial cells to culture the sample in the presence of a cell enlarger,
a second step of, after the first step, introducing an esterase substrate fluorescent stain into the culture medium,
a third step of isolating enlarged microbial cells stained in the second step according to a micromanipulation method to produce multiple culture media for establishment each inoculated with a single microbial cell,
a fourth step of culturing the microbial cell in each of the culture media for establishment; and
a fifth step of selecting culture media for establishment, in which cell proliferation has been confirmed, among the multiple culture media for establishment cultured in the fourth step.

2. The method according to Claim 1, wherein the cell enlarger is a pyridonecarboxylic acid-based antibiotic.

3. An agent for screening for efficiently obtaining useful microbial strains from a sample potentially containing multiple microbial cells, which contains a pyridonecarboxylic acid-based antibiotic as an active ingredient such that, under conditions where the agent is present in a suitable culture medium, cell division is inhibited to allow the microbial cells to enlarge and one of the enlarged microbial cells is transplanted to a culture medium for establishment to allow cell division to start.

4. A kit for screening for carrying out the method for screening defined in Claim 1 or 2, which comprises at least one of:
the culture medium used in the first step,
the cell enlarger used in the first step,
the esterase substrate fluorescent stain used in the second step; and
the culture media for establishment used in the third step.
